(19) **Europäisches Patentamt / European Patent Office / Office européen des brevets**

(11) **EP 4 497 986 B1**

(12) **FASCICULE DE BREVET EUROPEEN**

(45) Date de publication et mention
de la délivrance du brevet:
**12.11.2025 Bulletin 2025/46**

(21) Numéro de dépôt: **24191097.5**

(22) Date de dépôt: **26.07.2024**

(51) Classification Internationale des Brevets (IPC):
**F16L 33/22** (2006.01)  **F16L 37/127** (2006.01)
**F16L 37/084** (2006.01)  **B33Y 80/00** (2015.01)

(52) Classification Coopérative des Brevets (CPC):
**F16L 37/127; B33Y 80/00; F16L 33/22;
F16L 37/0844;** B29C 64/165; B33Y 10/00

(54) **ELÉMENT DE RACCORD ET PROCÉDÉ DE FABRICATION D'UN TEL ÉLÉMENT DE RACCORD**

VERBINDUNGSELEMENT UND VERFAHREN ZUR HERSTELLUNG EINES SOLCHEN
VERBINDUNGSELEMENTS

CONNECTING ELEMENT AND METHOD FOR MANUFACTURING SUCH A CONNECTING
ELEMENT

(84) Etats contractants désignés:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priorité: **27.07.2023 FR 2308107**

(43) Date de publication de la demande:
**29.01.2025 Bulletin 2025/05**

(73) Titulaire: **Staubli Faverges
74210 Faverges-Seythenex (FR)**

(72) Inventeur: **DURIEUX, Christophe
73200 Gilly sur Isere (FR)**

(74) Mandataire: **Lavoix
62, rue de Bonnel
69448 Lyon Cedex 03 (FR)**

(56) Documents cités:
WO-A2-2012/166546    FR-A1- 2 425 605
US-A- 2 191 579      US-A- 3 167 335

**Description**

La présente invention concerne un élément de raccord pour la connexion d'une canalisation de fluide à une durite. La présente invention concerne également un procédé de fabrication d'un tel élément de raccord.

Une technique connue pour connecter une conduite de fluide à une durite est de faire pénétrer une canule dans la durite, par déformation élastique de la durite. La canule définit un passage longitudinal qui est raccordé à la conduite. US3167335A divulgue un élément de raccord dans lequel une canule est disposée dans un corps équipé de moyens de mise en pression d'une durite. Ces moyens de mise en pression peuvent être neutralisés lors de la connexion et la déconnexion de la durite sur la canule. Ces moyens de mise en pression sont constitués par une mâchoire articulée autour d'un axe perpendiculaire et distant d'un axe longitudinal de la canule. La mâchoire est mobile entre une position de serrage, où elle presse la durite contre la canule, et une position de retrait, où elle est distante de la durite. Un ressort rappelle la mâchoire vers sa position de serrage. Un levier, solidaire de la mâchoire et accessible depuis l'extérieur du corps, permet à un opérateur d'amener la mâchoire dans sa position de retrait. Ce matériel ne permet pas d'assurer un maintien sûr et efficace de la durite sur la canule, notamment lorsque la pression du fluide circulant entre la conduite et la canule est importante. Une solution pour augmenter le serrage élastique de la durite sur la canule consiste à prévoir un diamètre extérieur de la canule très supérieur au diamètre intérieur de la durite, mais ceci rend plus difficile la connexion et la déconnexion de la durite et de la canule.

C'est à ces inconvénients qu'entend plus particulièrement remédier l'invention en proposant un nouvel élément de raccord pour la connexion rapide d'une canalisation de fluide et d'une durite dans lequel une mâchoire articulée sur le corps de l'élément de raccord peut être efficacement maintenue en position de serrage de la durite contre une canule.

A cet effet, l'invention a pour objet un élément de raccord pour la connexion d'une canalisation de fluide à une durite, cet élément de raccord comprenant une canule définissant un passage de fluide et s'étendant selon un axe longitudinal de l'élément de raccord, entre une partie avant configurée pour l'emmanchement de la durite et une partie arrière destinée à être raccordée à la canalisation de fluide ; un corps s'étendant selon l'axe longitudinal autour de, et solidairement à, la canule et définissant une embouchure d'accès à la partie avant de la canule ; au moins une première mâchoire, mobile en rotation, autour d'un premier axe de rotation, perpendiculaire à, et décalé de, l'axe longitudinal, entre

- ∘ une position de serrage dans laquelle la première mâchoire presse la durite contre la partie avant de la canule, dans une zone comprise, radialement à l'axe longitudinal, entre l'axe longitudinal et le premier axe de rotation et délimitée, le long de l'axe longitudinal, par un plan frontière, perpendiculaire à l'axe longitudinal et distant du premier axe de rotation d'une première distance, mesurée parallèlement à l'axe longitudinal, qui est non nulle, et
- ∘ une position de retrait dans laquelle la première mâchoire ne presse pas la durite contre la partie avant de la canule ;

un organe de manoeuvre, accessible depuis l'extérieur du corps, pour le déplacement de la première mâchoire, entre sa position de serrage et sa position de retrait.

Selon l'invention

- l'élément de raccord comprend

  - ∘ un poussoir, logé dans un volume interne du corps autour de la canule et mobile, en translation le long de l'axe longitudinal, par rapport au corps ;
  - ∘ un organe de rappel élastique du poussoir vers une position avancée ;

- le poussoir est pourvu d'une première surface d'appui contre la première mâchoire ;
- la première surface d'appui du poussoir est configurée pour exercer sur la première mâchoire un effort de déplacement de la première mâchoire de sa position de retrait vers sa position de serrage ;
- la première surface d'appui est disposée, par rapport à l'axe longitudinal, à l'opposé du premier axe de rotation ; et
- lorsque la première mâchoire est dans sa position de serrage, avec la première surface d'appui contre la première mâchoire, un premier écart radial, mesuré perpendiculairement à l'axe longitudinal, entre le premier axe de rotation et un point de contact de la surface de réception d'effort de la première mâchoire et de la première surface d'appui, a une valeur supérieure à la valeur de la première distance.

Grace à l'invention, le premier écart radial permet au poussoir de transmettre à la première mâchoire un effort reçu de l'organe de rappel élastique, avec un bras de levier plus important que le bras de levier de l'effort résistant exercé par la durite sur cette mâchoire. En d'autres termes, la géométrie des éléments constitutifs de l'élément de raccord assure une application efficace des efforts, ce qui fiabilise le serrage de la durite sur la canule. Ceci permet à la première mâchoire de résister efficacement à un effort de séparation entre la canule et la durite, notamment lorsque la pression du fluide traversant la canule et la durite est importante.

Selon des aspects avantageux mais non obligatoires de l'invention, un tel élément de raccord peut incorporer une ou plusieurs des caractéristiques suivantes, prise(s) selon toute combinaison techniquement admissible.

- L'organe de rappel élastique du poussoir vers sa position avancée est également un organe de renvoi de la première mâchoire vers sa position de serrage.
- Un rapport entre la valeur du premier écart radial et la valeur de la première distance est compris entre 1,5 et 10, de préférence entre 4 et 6, de préférence encore égal à 5.
- L'élément de raccord comprend également une deuxième mâchoire, mobile en rotation, autour d'un deuxième axe de rotation, perpendiculaire à, et décalé de, l'axe longitudinal, entre

  ◦ une position de serrage dans laquelle la deuxième mâchoire presse la durite contre la partie avant de la canule, dans une zone comprise, radialement à l'axe longitudinal, entre l'axe longitudinal et le deuxième axe de rotation et délimitée, le long de l'axe longitudinal, par un plan frontière, perpendiculaire à l'axe longitudinal et distant du premier axe de rotation d'une deuxième distance, mesurée parallèlement à l'axe longitudinal, qui est non nulle, et
  ◦ une position de retrait dans laquelle la deuxième mâchoire ne presse pas la durite contre la partie avant de la canule ;

  le poussoir comprend une deuxième surface d'appui contre la deuxième mâchoire, la deuxième surface d'appui du poussoir est configurée pour exercer sur la deuxième mâchoire un effort de déplacement de la deuxième mâchoire de sa position de retrait vers sa position de serrage, la deuxième surface d'appui est disposée, par rapport à l'axe longitudinal, à l'opposé du deuxième axe de rotation et, lorsque la deuxième mâchoire est dans sa position de serrage, avec la deuxième surface d'appui contre la deuxième mâchoire, un deuxième écart radial, mesuré parallèlement à l'axe longitudinal, entre le deuxième axe de rotation et un point de contact de la surface de réception d'effort de la deuxième mâchoire et de la deuxième surface d'appui, a une valeur supérieure à la valeur de la deuxième distance.
- Un rapport entre la valeur du deuxième écart radial et la valeur de la deuxième distance est compris entre 1,5 et 10, de préférence entre 4 et 6, de préférence encore égal à 5.
- Les valeurs des première et deuxième distances sont égales et les valeurs des premier et deuxième écarts radiaux sont égales.
- Le poussoir comprend un relief configuré pour exercer sur la deuxième mâchoire un effort de déplacement de la deuxième mâchoire, de sa position de serrage vers sa position de retrait.
- L'organe de manoeuvre de la première mâchoire est un levier solidaire de la première mâchoire.
- L'organe de manoeuvre de la première mâchoire est une bague mobile axialement, le long de l'axe longitudinal, par rapport au corps.

Selon un autre aspect, l'invention concerne un procédé de fabrication d'un élément de raccord tel que mentionné ci-dessus, plus particulièrement un procédé dans lequel

- le corps est pourvu d'au moins une surface radiale interne de guidage ;
- le poussoir est pourvu d'au moins une surface radiale externe de guidage configurée pour coopérer par engagement avec une surface radiale interne de guidage du corps pour guider le poussoir lors de ses déplacements selon l'axe longitudinal ;
- le poussoir est mobile, dans le passage du corps, jusqu'à une position de référence, dans laquelle la ou chaque surface radiale externe de guidage du poussoir est dégagée de toute surface radiale interne de guidage du corps et réciproquement ;
- le procédé comprend une étape a) consistant à réaliser par fabrication additive le corps et le poussoir simultanément, alors que le poussoir est dans sa position de référence.

Avantageusement, ce procédé comprend des étapes successives postérieures à l'étape a) et consistant à

b) déplacer le poussoir dans le corps de l'élément de raccord vers une position où les surfaces radiales externes de guidage sont engagées dans les surfaces radiales internes de guidage ;
c) pré-positionner les deux mâchoires dans un boitier ;
d) engager le boitier équipé des mâchoires dans le corps, à travers une ouverture ménagée dans le corps ;
e) mettre en place et visser des vis respectivement dans des paliers du boîtier et dans des orifices taraudés des mâchoires 10 et 12 de telle sorte que ces vis soient alignées sur le premier axe de rotation et sur le deuxième axe de rotation ;

f) monter l'organe de rappel élastique du poussoir dans le volume interne du corps, en appui contre le poussoir ;

g) mettre en place la canule à l'intérieur du poussoir et du corps, en amenant l'organe de rappel élastique en appui contre la canule.

L'invention sera mieux comprise et d'autres avantages de celle-ci apparaitront plus clairement à la lumière de la description qui va suivre de plusieurs modes de réalisation d'un élément de raccord conforme à l'invention et de son procédé de fabrication, donnée uniquement à titre d'exemple et faite en référence aux dessins annexés dans lesquels :

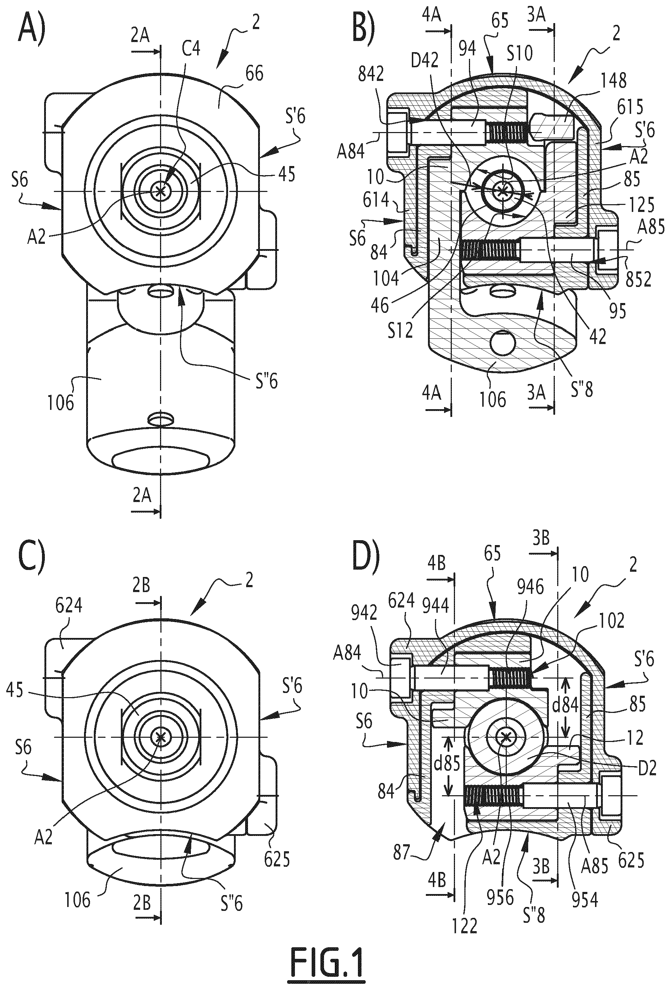

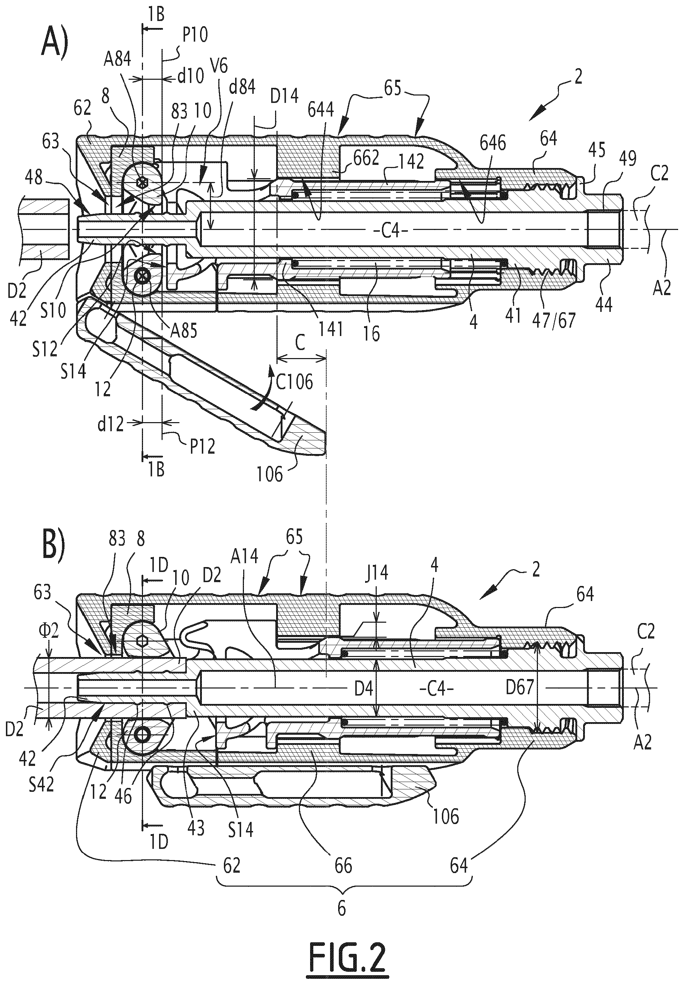

[Fig, 1] La figure 1 représente, sur quatre inserts A), B), C) et D), deux vues en élévation et deux coupes transversales d'un élément de raccord conforme à l'invention, dans deux configurations différentes. L'insert B) représente une coupe selon le plan 1B-1B visible sur l'insert A) de la figure 2. L'insert D) représente une coupe et selon le plan 1D-1D visible sur l'insert B) de la figure 2.

[Fig. 2] La figure 2 représente, sur deux inserts A) et B), deux coupes longitudinales de l'élément de raccord, prises respectivement selon le plan 2A-2A et selon le plan 2B-2B aux inserts A) et C) de la figure 1.

[Fig. 3] La figure 3 représente, sur deux inserts A) et B), deux coupes longitudinales du même élément de raccord, prises respectivement selon le plan 3A-3A et selon le plan 3B-3B sur les inserts B) et D) de la figure 1.

[Fig. 4] La figure 4 représente, sur deux inserts A) et B), deux coupes longitudinales du même élément de raccord, prises respectivement selon le plan 4A-4A et selon le plan 4B-4B sur les inserts B) et D) de la figure 1.

[Fig. 5] La figure 5 représente, sur deux inserts A) et B), l'élément de raccord en perspective et en vue éclatée, ainsi qu'un poussoir appartenant à l'élément de raccord, également en perspective.

[Fig. 6] La figure 6 représente, sur trois inserts A), B) et C) une vue en élévation et des coupes transversales d'un corps et d'un poussoir de l'élément de raccord des figures 1 à 5 en cours de fabrication, ainsi que, sur un insert D), une coupe longitudinale en perspective du corps, à plus petite échelle ;

[Fig. 7] La figure 7 représente, sur deux inserts A et B, deux coupes longitudinales du corps et du poussoir, prises respectivement selon le plan 7A-7A à l'insert A) de la figure 6 et selon la ligne de coupe 7B-7B à l'insert A) de la figure 7 ;

[Fig. 8] La figure 8 représente, sur deux inserts A) et B), deux coupes longitudinales analogues à celles de la figure 2, pour un élément de raccord conforme à un deuxième mode de réalisation de l'invention ;

[Fig. 9] La figure 9 représente, sur deux inserts A) et B), une coupe longitudinale analogue à celle de l'insert A) de la figure 2 pour un élément de raccord conforme à un troisième mode de réalisation et une vue en perspective éclatée du corps de cet élément de raccord, à plus petite échelle ; et

[Fig. 10] La figure 10 représente, sur deux inserts A) et B), deux coupes longitudinales analogues à celles de la figure 2 pour un élément de raccord conforme à un quatrième mode de réalisation de l'invention.

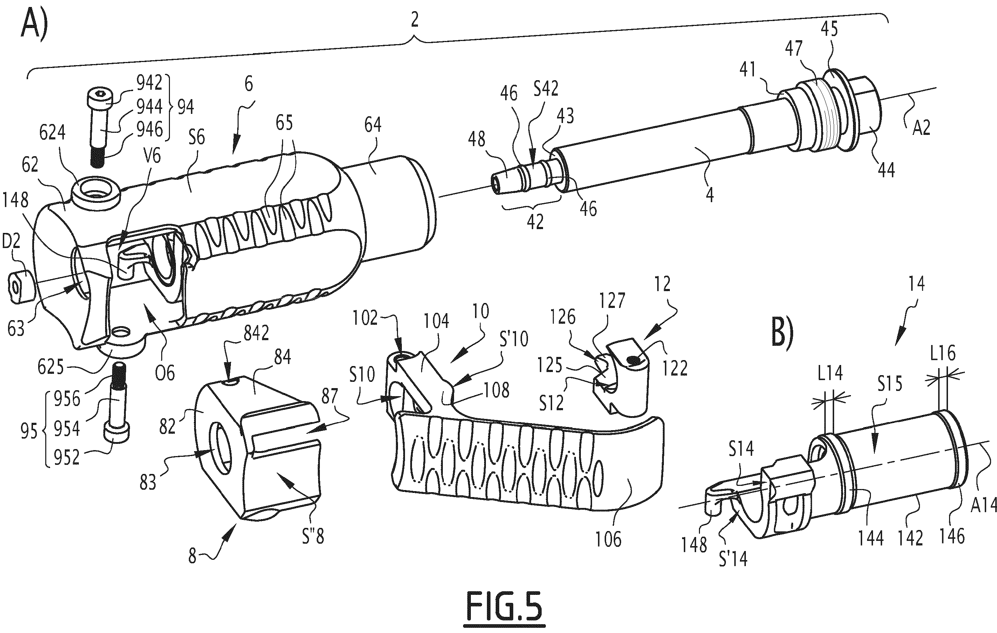

L'élément de raccord rapide 2 représenté sur les figures 1 à 5 est prévu pour être raccordé d'une part à une canalisation C2 et d'autre part à une durite D2. Pour la clarté du dessin, la canalisation C2 est représentée uniquement à la figure 2, en traits mixtes, et la durite D2 est représentée uniquement aux figures 1, 2, 4 et 5. Une durite est un type particulier de canalisation d'écoulement de fluide, qui a la particularité d'être souple et de pouvoir se dilater radialement. La durite D2 forme ici un élément de raccord complémentaire de l'élément de raccord 2.

Le fluide transitant dans la canalisation C2 et dans la durite D2 peut être un liquide ou un gaz, notamment avec une pression relativement élevée, par exemple supérieure ou égale à 1 MPa.

On définit le côté avant de l'élément de raccord 2 comme le côté tourné vers la durite D2 lors l'emmanchement de l'élément de raccord 2 et de la durite D2 ou lorsque ces éléments sont connectés. Sur les figures 2 à 5, le côté avant de l'élément de raccord 2 est orienté vers la gauche et son coté arrière est orienté vers la droite. L'élément de raccord 2 s'étend, entre ses cotés avant et arrière, selon un axe longitudinal A2.

Les vues en élévation de la figure 1 sont prises depuis l'arrière de l'élément de raccord 2.

L'élément de raccord comprend une canule 4 qui s'étend selon l'axe longitudinal A2 et qui présente une partie avant chanfreinée 42 et une partie arrière taraudée 44. La canule 4 définit un conduit C4 de passage de fluide entre ses parties avant et arrière 42 et 44.

On note D4 le diamètre externe de la canule 4 sur la plus grosse partie de sa longueur, entre ses parties avant et arrière. On note D42 le diamètre externe de la partie avant 42, qui est réduit par rapport au diamètre externe D4. La section de passage du conduit C4 présente, au niveau de la partie avant 42, une aire inférieure à son aire dans la partie intermédiaire et au niveau de la partie arrière 44.

La partie avant 42 de la canule 4 est pourvue de deux nervures périphériques 46 qui constituent des reliefs de retenue de la durite D2 sur cette partie.

La partie avant 42 porte un chanfrein 48 convergeant vers l'avant et dont la fonction est de faciliter l'introduction de la canule 4 dans la durite D2.

La partie arrière 44 est équipée d'un taraudage interne 49 de montage de la conduite C2.

Dans l'exemple des figures, la canule 4 est monobloc. En variante non représentée, elle peut être constituée de plusieurs pièces assemblées de façon étanche les unes avec les autres.

A proximité de sa partie arrière 44, la canule 4 comprend une collerette 45 et un filetage externe 47.

L'élément de raccord 2 comprend également un corps 6 qui est monobloc et qui s'étend le long de l'axe longitudinal A2, entre une extrémité avant 62 et une extrémité arrière 64. Vu à partir de la canalisation C2, l'extrémité arrière 64 est une extrémité proximale et l'extrémité avant 62 est une extrémité distale du corps 6.

Au niveau de son extrémité avant ou distale 62, le corps 6 définit une embouchure 63 centrée sur l'axe longitudinal A2 et à travers laquelle fait saillie la partie avant 42 de la canule 4, vers l'avant de l'élément de raccord 2. L'embouchure 63 donne accès à la partie avant 42 de la canule.

On note D63 le diamètre de l'embouchure 63.

On note V6 le volume interne du corps 6. La canule est, pour l'essentiel reçue dans le volume interne V6, sauf la portion de sa partie avant 42 en saillie à travers l'embouchure 63.

Au niveau de son extrémité arrière 64, le corps 4 est pourvu d'un taraudage 67 qui coopère avec le filetage 47 de la canule 4 pour visser la canule 4 dans le corps 6 jusqu'à ce que la collerette 45 vienne en appui contre l'extrémité arrière ou proximale 64, ce qui solidarise les éléments 4 et 6 et positionne la canule 4 par rapport au corps 6, le long de l'axe longitudinal A2.

On note D67 le diamètre du taraudage 67. Ce taraudage définit une ouverture proximale de passage de la canule 4 dans le corps 6.

On note 66 la partie intermédiaire du corps 6 définie, le long de l'axe A2 entre ses extrémités avant et arrière 62 et 64. Au niveau de cette partie intermédiaire 66, le corps 62 présente une géométrie extérieure globalement en forme de cylindre tronqué par deux plans, parallèles aux plans des figures 2 à 4 et qui définissent deux surfaces latérales S6 et S'6 disposées de part et d'autre du plan de coupe de l'insert A) de la figure 2. On note respectivement 614 et 615 les parois latérales globalement planes du corps 6 qui définissent, sur l'extérieur, les surfaces S6 et S'6.

Le corps 6 définit également une surface externe concave S"6 qui est située entre les surfaces S6 et S'6, en partie inférieure du corps 6 dans la position des figures 1à 4.

Entre les surfaces S6, S'6 et S"6, le corps 6 porte des stries externes 65 qui facilitent sa prise en main.

Du côté de la surface concave S"6 et sur l'avant de celle-ci, le corps 6 définit une ouverture O6 de forme globalement rectangulaire et à travers laquelle un boitier 8 est engagé dans le volume interne V6 du corps 6.

Ce boitier 8 comprend une face avant 82 qui est perpendiculaire à l'axe longitudinal A2 en configuration montée du boitier 8 dans le corps 6 et dans laquelle est ménagé un orifice circulaire 83 qui est aligné avec l'embouchure 63 dans cette configuration. L'orifice 83 permet le passage de la partie avant 42 de la canule 4 et de la durite D2.

Le boitier 8 comprend deux parois latérales 84 et 85 qui s'étendent parallèlement l'une à l'autre à partir d'un couvercle 86. Le couvercle 86 obture l'ouverture O6 en configuration montée du boitier 8 dans le corps 6 et définit une surface concave S"8 qui est affleurante avec la surface S"6 du corps 6 dans cette configuration.

Une encoche 87 est prévue dans le couvercle 86 et débouche sur le côté arrière du boitier 8.

En configuration montée du boitier 8 dans le corps 6, les parois latérales 84 et 85 s'étendent parallèlement aux parois latérales 614 et 615, donc parallèlement aux surfaces S6 et S'6. De préférence, les parois latérales 84 et 85 s'étendent respectivement le long des parois latérales 614 et 615, dans le volume interne V6, donc sur l'intérieur des parois latérales 614 et 615.

La paroi latérale 84 est percée d'un orifice traversant 842 ménagé au niveau d'une portion en surépaisseur de la paroi latérale 84 et qui définit un palier centré sur un axe A84 perpendiculaire à l'axe longitudinal A2 et distant de celui-ci d'une distance d84, mesurée perpendiculairement aux axes A2 et A84, qui est non nulle. De la même façon, la paroi latérale 85 est percée d'un orifice traversant 852 ménagé au niveau d'une portion en surépaisseur de la paroi 85. L'orifice traversant 852 définit un palier centré sur un axe A85. L'axe A85 est perpendiculaire à l'axe A2 et situé à une distance non nulle d85 de l'axe A2, cette distance d85 étant mesurée perpendiculairement aux axes A2 et A85.

Avantageusement, et comme représenté sur les figures, les axes A84 et A85 sont parallèles et les distances d84 et d85 sont égales. Ainsi, dans un plan transversal de l'élément de raccord 2, c'est-à-dire un plan perpendiculaire à l'axe longitudinal A2, les axes A84 et A85 sont symétriques par rapport à l'axe longitudinal A2. Ceci n'est toutefois pas obligatoire.

Le corps 6 est pourvu, au niveau de ses surfaces 614 et 615, de deux sièges 624 et 625 qui sont respectivement alignés avec les orifices traversant 842 et 852 et centrés sur les axes A84 et A85 en configuration montée du boitier 8 dans le corps 6.

L'élément de raccord 2 comprend également une première mâchoire 10 et une deuxième mâchoire 12.

La première mâchoire 10 est articulée autour de l'axe A84 sur le corps 6 équipé du boitier 8. Pour ce faire, une première vis décolletée 94 est insérée dans le palier formé par l'orifice traversant 842 en traversant le siège 624 et en étant vissée dans la mâchoire 10. La tête 942 de la vis 94 est reçue dans le siège 624, alors que sa tige 944 traverse l'orifice 842 et est vissée, par une partie filetée 946, dans un orifice taraudé 102 de la mâchoire 10.

De façon comparable, la deuxième mâchoire 12 est articulée autour de l'axe A85 au moyen d'une vis décolletée 95 dont la tête 952 est reçue dans le siège 625, dont la tige 954 traverse l'orifice 852 et est vissée, par une partie filetée 956, dans un orifice taraudé 122 de la mâchoire 12.

Chacune des première et deuxième mâchoires 10 et 12 est mobile, respectivement autour de l'axe A84 ou de l'axe A85, par rapport au corps 6 équipé du boitier 8, entre une position de serrage et une position de retrait, autrement dit une position relâchée. L'axe A84 est ainsi un premier axe de rotation de la première mâchoire 10, alors que l'axe A85 est un deuxième axe de rotation de la deuxième mâchoire 12. Dans sa position de serrage, si la durite D2 est emmanchée sur la canule 4, la mâchoire 10 ou 12 presse la durite contre la surface radiale externe S42 de la partie avant 42 de la canule. Dans sa position de retrait, la mâchoire 10 ou 12 ne presse pas la durite contre la surface radiale externe de partie avant de la canule.

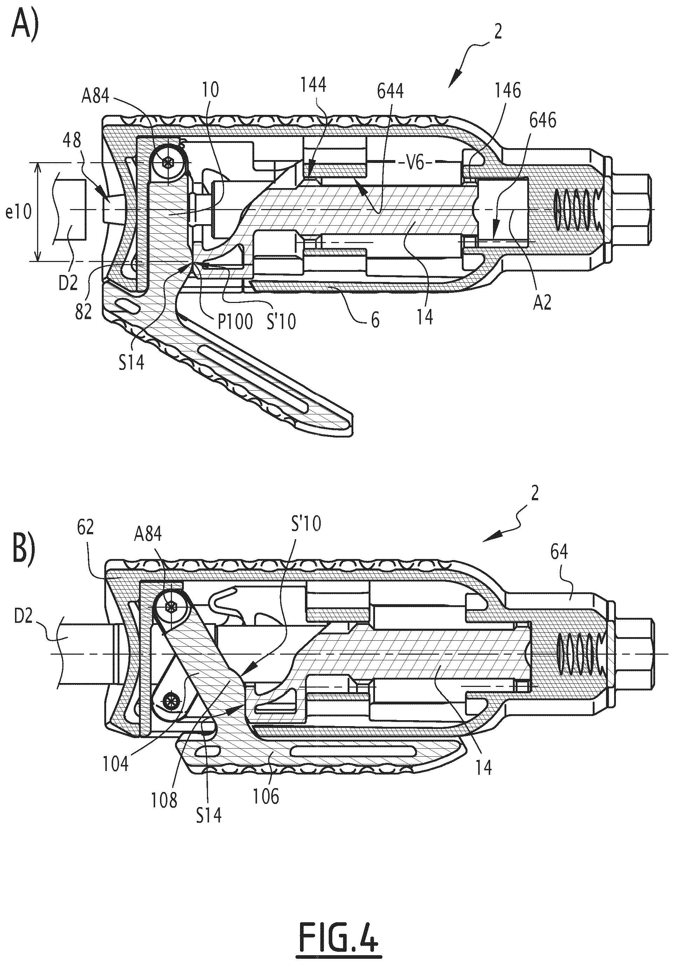

La position de serrage des mâchoires 10 et 12 est représentée sur les inserts A) et B) de la figure 1 ainsi que sur les inserts A) des figures 2 à 4, la durite étant représentée à l'extérieur de l'élément de raccord 2 sur l'insert A) des figures 2 et 4. La position de retrait des mâchoires 10 et 12 est représentée sur l'insert D) de la figure 1, ainsi que sur les inserts B) des figures 2 et 4, la durite D2 étant représentée en position non serrée sur la partie avant 42 de la canule 4 sur l'insert B) des figures 2 et 4.

La première mâchoire 10 définit une surface S10 d'appui sur la durite D2, lorsque la première mâchoire 10 est dans sa position de serrage et lorsque la durite est emmanchée sur la canule. La deuxième mâchoire S12 définit également une surface S12 d'appui contre la durite D2 lorsque la deuxième mâchoire 12 est dans sa position de serrage et lorsque la durite est emmanchée sur la canule.

Chaque surface d'appui S10 ou S12 présente un profil progressif vis-à-vis de la partie avant 42 de la canule 4 qui est tel que, quand la mâchoire 10 ou 12 est en position de serrage, la distance entre l'axe longitudinal A2 et une projection orthogonale de cette surface d'appui S10 ou S12 sur un plan perpendiculaire à l'axe longitudinal est inférieure à la moitié du diamètre extérieur $\Phi 2$ de la durite D2 libre et emmanchée sur la partie avant 42 de la canule 4, alors que, quand la mâchoire 10 ou 12 est en position de retrait, la distance entre l'axe longitudinal A2 et la projection de la surface d'appui S10 ou S12 sur le même plan perpendiculaire est supérieure à la moitié du diamètre extérieur $\Phi 2$.

La zone définie par la surface d'appui S10, dans laquelle la première mâchoire 10 presse la durite D2 contre la partie avant 42 de la canule 4 dans la position de serrage de la première mâchoire, est comprise, radialement à l'axe longitudinal A2, entre cet axe longitudinal et le premier axe de rotation A84. Dans la position de serrage, la zone dans laquelle la première mâchoire 10 presse la durite D2 est limitée, vers l'arrière c'est-à-dire à l'opposé du premier axe de rotation A84 le long de l'axe longitudinal A2, par un plan frontière P10 qui est perpendiculaire à l'axe longitudinal A2 et qui passe par le point de la surface d'appui S10 le plus éloigné du premier axe de rotation A84 selon une direction parallèle à cet axe longitudinal. On note d10 la distance, mesurée parallèlement à l'axe longitudinal A2, entre le premier axe de rotation A84 et le plan frontière P10.

La distance d10 est le bras de levier maximum de l'effort résistant exercé par la durite D2 sur la première mâchoire 10 en position de serrage, lorsque la durite est emmanchée sur la canule 4.

La zone définie par la surface d'appui S12, dans laquelle la deuxième mâchoire 12 presse la durite D2 contre la partie avant 42 de la canule 4 dans la position de serrage de la deuxième mâchoire, est comprise, radialement à l'axe longitudinal A2, entre cet axe longitudinal et le deuxième axe de rotation A85. Dans la position de serrage, la zone dans laquelle la deuxième mâchoire 12 presse la durite D2 est limitée, vers l'arrière c'est-à-dire à l'opposé du deuxième axe de rotation A85 le long de l'axe longitudinal A2, par un plan frontière P12 qui est perpendiculaire à l'axe longitudinal A2 et qui passe par le point de la surface d'appui S12 le plus éloigné du deuxième axe de rotation A85 selon une direction parallèle à cet axe longitudinal. On note d12 la distance, mesurée parallèlement à l'axe longitudinal A2, entre le deuxième axe de rotation A85 et le plan frontière P12.

La distance d12 est le bras de levier maximum de l'effort résistant exercé par la durite D2 sur la deuxième mâchoire 12 en position de serrage, lorsque la durite est emmanchée sur la canule 4.

Dans l'exemple des figures, les axes de rotation A84 et A85 sont disposés au même niveau le long de l'axe longitudinal A2, de même que les plans de frontière P10 et P12. Ainsi, les distances d10 et d12 sont égales.

Ceci n'est toutefois pas obligatoire et d'autres répartitions spatiales des premier et deuxième axes de rotation A84 et A85 et des premier et deuxième plans frontière P10 et P12 sont envisageables.

La première mâchoire 10 comprend un bras latéral 104 qui s'étend radialement au premier axe de rotation A84 et qui définit une surface S'10 de réception d'un effort. La surface de réception d'effort S'10 est ménagée sur l'arrière du bras latéral 104 et à l'opposé du premier axe de rotation A84 par rapport à l'axe longitudinal A2. En d'autres termes, dans un plan parallèle à l'axe longitudinal A2 et perpendiculaire au premier axe de rotation A84, le premier axe de rotation A84 et la surface de réception d'effort S'10 sont situés de part et d'autre de l'axe longitudinal A2, comme montré à la figure 4.

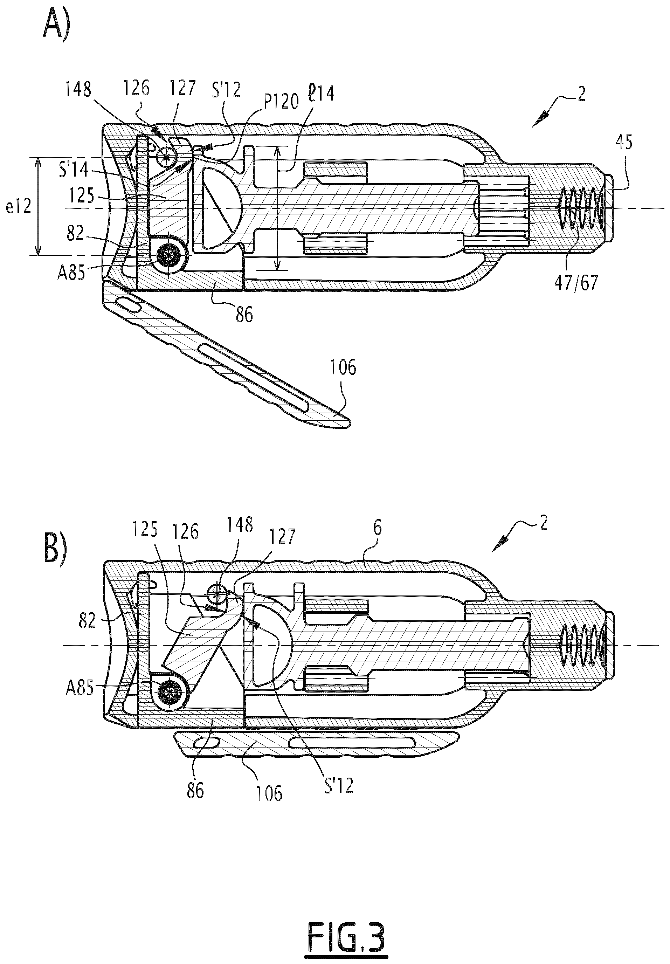

La deuxième mâchoire 12 comprend également un bras latéral 125 qui s'étend radialement au deuxième axe de rotation A85 et qui définit une surface S'12 de réception d'un effort. La surface de réception d'effort S'12 est ménagée sur l'arrière du bras latéral 125. Dans un plan parallèle à l'axe longitudinal A2 et perpendiculaire au deuxième axe de rotation A85, le deuxième axe de rotation A85 et la surface de réception d'effort S'12 sont disposés de part et d'autre de l'axe longitudinal A2, comme montré à la figure 3.

Le bras latéral 125 est pourvu, à son extrémité opposée à l'orifice taraudé 122, d'un crochet 127 qui définit un renfoncement concave 126. La surface de réception d'effort S'12 est ménagée sur l'extérieur du crochet 127, à l'opposé du renfoncement concave 126.

En configuration assemblée de l'élément de raccord 2, les bras latéraux 104 et 125 sont respectivement adjacents aux parois latérales 84 et 85 du boitier 8 et disposés sur l'intérieur de ces parois, vis-à-vis desquelles ils peuvent pivoter, respectivement autour des axes de rotation A84 et A85.

La première mâchoire comprend également un organe de manoeuvre formé par un levier 106 qui dépasse du corps 6 et du boîtier 8 à travers l'encoche 87. Ce levier 106 est monobloc avec la portion de la première mâchoire 10 dans laquelle sont ménagés l'orifice taraudé 102, la surface d'appui S10, ainsi qu'avec le bras latéral 104, donc avec la surface de réception d'effort S'10.

Le levier 106 permet de manoeuvrer la première mâchoire 10 entre sa position de serrage et sa position de retrait, en plaquant le levier 106 en direction des surfaces S"6 et S"8, ce qui le fait pivoter autour du premier axe de rotation A84, dans le sens trigonométrique à la figure 2. La position de serrage de l'insert A) des figures 2 à 4 est une position par défaut prise par la mâchoire 10 si aucun effort n'est exercé sur le levier 106 par un utilisateur de l'élément de raccord 2.

L'élément de raccord 2 comprend également un poussoir 14 qui s'étend le long d'un axe longitudinal A14 confondu avec l'axe longitudinal A2 en configuration montée de l'élément de raccord 2.

Le poussoir 14 est monté à l'intérieur du corps 6, dans le volume interne V6 et autour de la canule 4, entre ses parties avant et arrière 42 et 44 le long de l'axe longitudinal A2, en étant soumis à l'action d'un organe élastique, dans l'exemple formé par un ressort spiral 16, qui tend à repousser le poussoir 14 vers l'avant, c'est-à-dire en direction de l'extrémité avant 62 du corps 6. La position avancée du poussoir 14, représentée sur les inserts A) des figures 2 à 4, est une position par défaut du poussoir, prise par celui-ci sous l'action du ressort spiral 16 tant qu'un utilisateur ne rabat pas le levier 106 vers les surfaces S"6 et S"8.

En variante, le ressort 16 peut être remplacé par un autre organe élastique de renvoi du poussoir vers l'avant du corps 6.

Le poussoir 14 est prévu pour se déplacer longitudinalement, c'est-à-dire en translation parallèlement à l'axe longitudinal A2, par rapport au corps 6, au boitier 8 et aux mâchoires 10 et 12. Le poussoir forme donc un coulisseau vis-à-vis du corps 6, du boîtier 8 et des mâchoires 10 et 12.

Le poussoir 14 comprend une jupe annulaire 142 centrée sur l'axe A14 et pourvue, à chacune de ses extrémités, d'une surface radiale externe de guidage, à savoir, une surface radiale externe de guidage avant 144 et une surface radiale externe de guidage arrière 146.

En pratique, les surfaces radiales externes de guidage 144 et 146 sont à section transversale circulaire. Ainsi, les surfaces externes de guidage sont chacune inscrites dans un cylindre à base circulaire, de diamètre D14 ou D16.

On note D14 et D16 leurs diamètres qui, de préférence, ont la même valeur.

En variante non-représenté de l'invention, les diamètres D14 et D16 ont des valeurs différentes.

On note L14 la longueur axiale de la surface radiale externe de guidage avant 144 et L16 la longueur axiale de la surface radiale externe de guidage arrière 146, ces longueurs axiales étant mesurées parallèlement à l'axe longitudinal A14.

Dans l'exemple, et selon un aspect avantageux de l'invention, les longueurs L14 et L16 sont égales.

En variante non représentée de l'invention, elles peuvent être différentes.

On note S15 la surface radiale externe de la jupe 142 située, le long de l'axe longitudinal A14, entre les surfaces radiales externes de guidage 144 et 146 qui sont espacées l'une de l'autre le long de l'axe longitudinal A2. On note D15 le diamètre de la surface S15.

Ce diamètre D15 est strictement inférieur aux diamètres D14 et D16. En d'autres termes, la surface S15 définit une surface de jonction cylindrique dont le diamètre D15 est inférieur au diamètre D14 de la surface radiale externe de guidage avant 144 et au diamètre D16 de la surface radiale externe de guidage arrière 146.

Le corps 6 est, quant à lui, pourvu d'une surface radiale interne de guidage avant 644 et d'une surface radiale interne de guidage arrière 646, toutes deux centrées sur l'axe longitudinal A2. La surface radiale interne de guidage avant 644 est ménagée au centre d'une nervure périphérique interne 662 de la partie intermédiaire 66 du corps 6. La surface radiale interne de guidage arrière 644 est ménagée à la jonction entre les parties intermédiaire 66 et arrière 64 du corps 6.

On note respectivement L64 et L66 la longueur axiale des surfaces radiales internes avant et arrière 644 et 646, ces longueurs axiales étant mesurées parallèlement à l'axe longitudinal A2.

La surface radiale interne avant 644 est pourvue de cannelures 648 qui s'étendent sur toute sa longueur L64, alors que la surface radiale interne de guidage arrière 646 est pourvue de cannelures 650 qui s'étendent sur toute sa longueur L66.

Dans un plan radial perpendiculaire à l'axe longitudinal A2, les sommets des nervures qui séparent les cannelures 648 définissent un cercle inscrit dans la surface radiale interne de guidage avant 644, dont on note D64 le diamètre. De la même façon, dans un plan radial à l'axe longitudinal A2, les sommets des nervures qui séparent les cannelures 650 définissent un cercle inscrit à la surface radiale interne de guidage arrière 646, dont on note D66 le diamètre. Ainsi, les surfaces internes de guidage sont chacune inscrites autour d'un cylindre à base circulaire, de diamètre D64 ou D66.

Les diamètres D64 et D66 ont avantageusement la même valeur, comme les diamètres D14 et D16.

En variante non-représenté de l'invention, les diamètres D64 et D66 ont des valeurs différentes.

Dans tous les cas, les diamètres D14, D64, D16 et D66 sont choisis pour que les surfaces radiales de guidage 144 et 644, d'une part 146 et 646 d'autre part, guident efficacement le poussoir 14 en translation le long de l'axe A2, à l'intérieur du corps 6, lors de son coulissement vis-à-vis du corps 6.

Le rapport des diamètres D14 et D64 et le rapport des diamètres D16 et D66 sont tels qu'un jeu de guidage J14 d'épaisseur radiale relativement faible, par exemple de l'ordre de quelques dixièmes de millimètres, est ménagé entre les surfaces radiales externes et internes de guidage 144 et 644, d'une part, 146 et 646, d'autre part. En d'autres termes, les diamètres D14, D16, D64 et D66 sont égaux, au jeu de guidage J14 près.

On note C la course du poussoir 14 sous l'action du ressort 16. Cette course correspond au passage du poussoir 14 de la position de l'insert B) sur l'une des figures 2 à 4, qui est une position arrière du poussoir 14 par rapport au corps 6, à la position de l'insert A) sur ces figures, qui est une position avant du poussoir 14 par rapport au corps 6, ou réciproquement au passage de la position de l'insert A) à celle de l'insert B).

La longueur L64 est supérieure ou égale à la somme de la longueur L14 et de la course C. De la même façon, la longueur L66 est supérieure ou égale à la somme de la longueur L16 et de la course C.

On a les relations suivantes :

$$L64 \geq L14 + C \qquad\qquad (\text{équation 1})$$

$$L66 \geq L16 + C \qquad\qquad (\text{équation 2})$$

Grâce à ces relations entre les longueurs L14 et L64, L16 et L66 et la course C, en cours de fonctionnement de l'élément de raccord 2, la surface radiale externe de guidage avant 144 reste en regard de la surface radiale interne de guidage avant 644 et la surface radiale externe de guidage arrière 146 reste en regard de la surface radiale interne de guidage arrière 646, le long de l'axe longitudinal A2.

Sur son côté avant, le poussoir 14 définit une première surface d'appui S14 qui est perpendiculaire à l'axe A14 et qui vient en butée contre la surface de réception d'effort S'10 de la première mâchoire 10, sous l'action du ressort de rappel 16, pour autant que le levier 106 n'est pas rabattu vers le corps 6, c'est-à-dire lorsque la première mâchoire 10 est par défaut dans sa position de serrage. En d'autres termes, par défaut, l'effort élastique du ressort 16 est transmis au poussoir 14 qui exerce, par sa première surface d'appui S14 et sur la surface de réception d'effort S'10, un effort axial dirigé vers l'avant, lequel a pour effet de ramener la première mâchoire 10 dans sa position de serrage.

Ainsi, le ressort de rappel 16, qui est un organe de rappel élastique du poussoir 14 vers sa position avancée, est également un organe de renvoi de la première mâchoire 10 vers sa position de serrage.

Le poussoir 14 est orienté autour de l'axe A2 de telle sorte que sa surface d'appui S14 est située, dans un plan perpendiculaire au premier axe de rotation A84, du côté de l'axe longitudinal A2 opposé à ce premier axe de rotation 84. On note e10 un écart radial, mesuré perpendiculairement à l'axe longitudinal A2, dans un plan perpendiculaire au premier axe de rotation A84, entre le premier axe de rotation A84 et le point de contact P100 de la surface de réception d'effort S'10 de la première mâchoire 10 et de la surface S14 dans ce plan perpendiculaire au premier axe de rotation A84. Ce plan perpendiculaire au premier axe de rotation A84 est, dans l'exemple des figures, celui de la figure 4, où l'écart radial e10 est repéré.

Cet écart radial e10 est le bras de levier par rapport à l'axe de rotation A84 de l'effort transmis entre les surfaces S14 et S'10 lorsque la première mâchoire 10 est dans sa position de serrage. Cet écart radial e10 a une valeur supérieure à la valeur de la première distance d10.

Ceci provient notamment du fait que la surface de réception d'effort S'10 est ménagée sur la tranche d'une bosse 108 définie par le bras latéral 104, à l'opposé de l'orifice taraudé 102 par rapport à un plan parallèle au premier axe de rotation A84 et contenant l'axe longitudinal A2.

Ainsi, le bras de levier, autour du premier axe de rotation A84, de l'effort transmis entre les surfaces S14 et S'10 est supérieur au bras de levier maximum de l'effort de serrage exercé sur la durite 2 par la surface d'appui S10 de la première mâchoire 10. L'effort transmis entre les surfaces S14 et S'10 est donc amplifié par le rapport de la valeur de l'écart radial e10 sur la valeur de la distance d10. Il est donc garanti que la première mâchoire 10 est efficacement maintenue en position de serrage par le poussoir 14 et le ressort 16, malgré l'effort résistant exercé par la durite D2.

Avantageusement, un rapport entre la valeur du premier écart axial e10 et la valeur de la première distance d10 est compris entre 1,5 et 10, de préférence entre 4 et 6, de préférence encore égal à 5.

Le poussoir 14 définit également une deuxième surface d'appui S'14 qui, dans l'exemple des figures, est coplanaire avec la première surface d'appui S14, même si cela n'est pas obligatoire.

La deuxième surface d'appui S'14 est, de préférence, perpendiculaire à l'axe longitudinal A14 et elle est configurée pour venir en butée contre la surface de réception d'effort S'12 de la deuxième mâchoire 12, comme visible à la figure 3.

On note e12 un écart radial, mesuré perpendiculairement à l'axe longitudinal A12 et dans un plan perpendiculaire au deuxième axe de rotation A85, entre le deuxième axe de rotation A85 et le point de contact P120 de la surface de réception d'effort S'12 de la deuxième mâchoire 12 et de la surface S'14. Ce plan perpendiculaire au deuxième axe de rotation A85 est, dans l'exemple des figures, celui de la figure 3, où l'écart e12 est repéré.

La surface S'14 exerce, sous l'action du ressort 16, un effort d'appui sur la surface de réception d'effort S'12 qui renvoie par défaut la deuxième mâchoire vers sa position de serrage.

L'écart radial e12 est le bras de levier par rapport à l'axe de rotation A85 de l'effort transmis entre les surfaces S'14 et S'12 lorsque la deuxième mâchoire 12 est dans sa position de serrage. Cet écart radial e12 a une valeur supérieure à celle de la distance d12.

Ainsi, le bras de levier, autour du deuxième axe de rotation A85, de l'effort transmis entre les surfaces S'14 et S'12 est supérieur au bras de levier maximum de l'effort de serrage exercé sur la durite 2 par la surface d'appui S12 de la deuxième mâchoire 12. L'effort transmis entre les surfaces S'14 et S'12 est donc amplifié par le rapport de la valeur l'écart radial e12 sur la valeur la distance d12. Il est donc garanti que la deuxième mâchoire 12 est efficacement maintenue en position de serrage par le poussoir 14 et le ressort 16, malgré l'effort résistant exercé par la durite D2.

Le poussoir 14 comprend également un doigt 148 qui est engagé dans le renfoncement 126 de la deuxième mâchoire 12. Le doigt 148 constitue un relief du poussoir 14 destiné à interagir avec la deuxième mâchoire 12. En variante, le doigt 148 peut être remplacé par un autre relief. Qu'il s'agisse du doigt ou d'un autre relief, il exerce sur la deuxième mâchoire 12 un effort de déplacement de cette mâchoire de sa position de serrage vers sa position de retrait, comme expliqué ci-après.

Lorsqu'un utilisateur exerce sur le levier 106 un couple C106 autour du premier axe de rotation A84, qui a pour effet de faire passer ce levier donc l'ensemble de la première mâchoire 10 de la position des inserts A) à la position des inserts B) aux figures 2 à 4, le bras 104 de la première mâchoire 10, qui est solidaire du levier 106, exerce par sa bosse 108 un effort qui repousse le poussoir 14 en direction de l'extrémité arrière 64 du corps 6. En d'autres termes, la surface S'10 constitue alors une surface d'appui contre la surface S14 qui reçoit l'effort exercé par la mâchoire 10, cet effort ayant une composante axiale parallèle à l'axe longitudinal A2 et dirigée vers l'arrière, qui s'oppose à l'effort élastique du ressort 16.

Si le couple exercé C106 est suffisamment intense, le poussoir 14 recule vers l'arrière sous l'action de l'effort transmis entre les surfaces S'10 et S14, ce qui est visible par la comparaison des inserts B) aux inserts A) sur les figures 2 à 4. C'est ce qui permet de ramener la mâchoire 10 dans sa position de retrait représentée sur les inserts B) des figures 2 à 4.

Le doigt 148 du poussoir 14 recule avec le reste de ce poussoir, ce qui a pour effet d'exercer sur la surface concave du crochet 127, dans le renfoncement 126, un effort d'entrainement de la mâchoire 12 vers l'extrémité arrière 64 du corps 6. Ceci déplace la deuxième mâchoire 12 vers sa position de retrait représentée sur les inserts B) des figures 2 à 4.

Ainsi, la manoeuvre du levier 106, qui consiste à le rabattre en direction des surfaces S"6 et S"8, a pour effet de déplacer les deux mâchoires 10 et 12 de leurs positions de serrage à leurs positions de retrait respectives.

L'atteinte de ces positions de retrait permet d'introduire la durite D2 sur la partie avant 42 de la canule 4, sans être gênée par les mâchoires 10 et 12.

Il est alors possible pour l'utilisateur de relâcher le levier 106 qui n'exerce alors plus de couple sur la première mâchoire, autour du premier axe de rotation A84, de sorte que le poussoir 14 soumis à l'action du ressort 16 repousse efficacement les deux mâchoires 10 et 12 vers leurs positions de serrage respectives, au moyen de ses surfaces d'appui S14 et S'14 qui agissent respectivement sur les surfaces de réception d'effort S'10 et S'12.

Compte tenu du rapport des valeurs de e10/d10 et e12/d12, l'effort élastique exercé par le ressort 16 est amplifié et efficace pour ramener puis maintenir les deux mâchoires 10 et 12 en position de serrage, malgré l'effort résistant exercé sur les surfaces d'appui S10 et S12 par la surface radiale externe de la durite D2.

On note ℓ14 la largeur maximale du poussoir 14 mesurée perpendiculairement à l'axe longitudinal A14. Cette largeur définit l'encombrement radial du poussoir 14, c'est-à-dire le diamètre minimum d'un cercle à travers lequel peut passer le poussoir 14.

La largeur ℓ14 est strictement supérieure aux diamètres D63 et D67.

Ainsi, le poussoir ne peut pas être introduit dans le corps 6, ou sortir de celui-ci, à travers ses extrémités avant 62 et arrière 64.

Dans un but d'optimisation de la gamme de fabrication de l'élément de raccord 2, les composants 6 et 14 sont réalisés simultanément par fabrication additive par jet de liant, parfois dénommé « usinage additif par jet de liant ». Le principe de l'usinage additif par jet de liant consiste à déposer une fine couche de matériau en poudre, puis à appliquer un liant qui va agglomérer les grains de poudre exposés à de la chaleur ou à de la lumière. La source de chaleur ou de lumière est mobile dans un plan et permet l'agglomération des grains correspondant à une tranche des pièces. La production des pièces se fait donc par tranches successives parallèlement à un plan de pose initial. Dans le cas de l'usinage simultané du corps 6 et du poussoir 14, le plan de pose initial peut être choisi comme le plan perpendiculaire à l'axe longitudinal A2 et situé au bout de l'extrémité arrière 64 du corps. Le corps 6 et le poussoir 14 sont réalisés tranche par tranche simultanément sans qu'il soit nécessaire de prévoir une liaison entre ces deux pièces. Elles sont maintenues en place par les grains de poudre qui n'ont pas été agglomérés. Une fois le corps 6 et le poussoir 14 produits, il est nécessaire d'évacuer les grains de poudre qui n'ont pas été agglomérés.

Le corps 6 et le poussoir 14 sont réalisés dans le même matériau. Par exemple, la poudre peut être un polyamide et le liant activé par de la chaleur.

La fabrication additive permet de réaliser, en une opération, une première pièce creuse dans laquelle est disposée une deuxième pièce, comme le corps 6 dans lequel est disposé le poussoir 14, sans limitation sur la mise en place de la deuxième pièce au sein de la première pièce creuse.

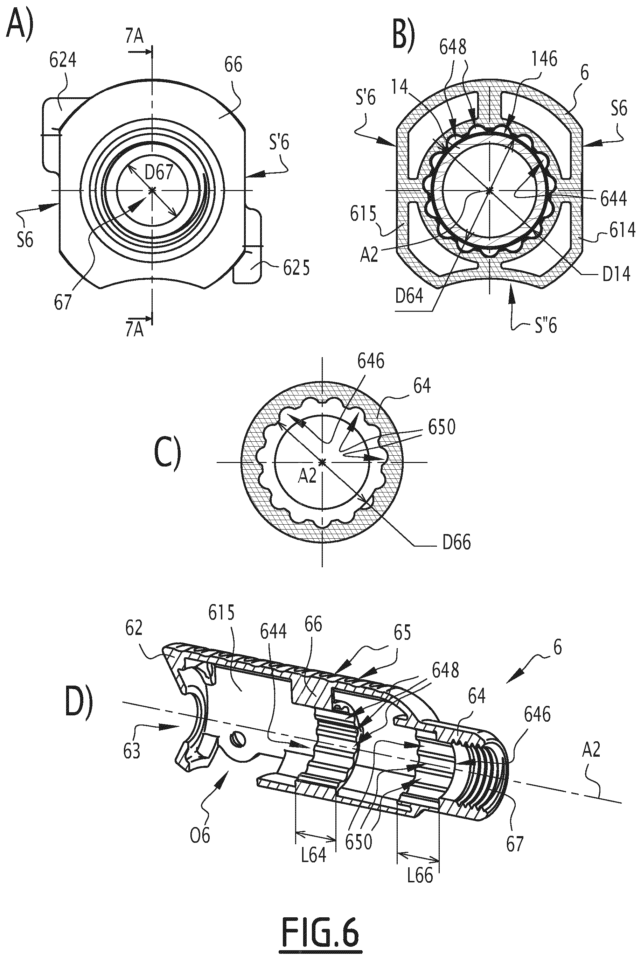

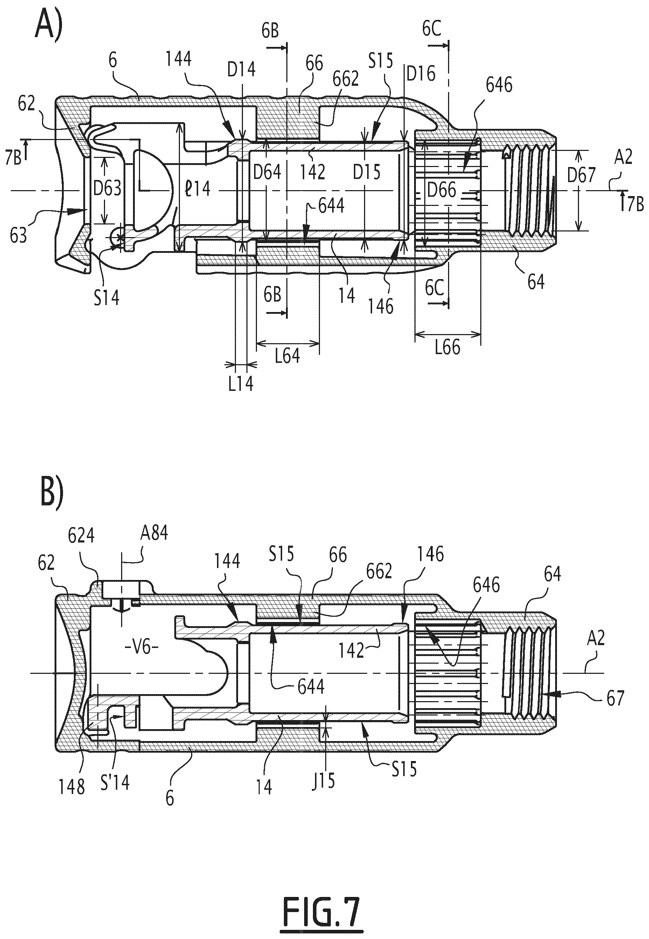

Pour permettre une telle fabrication additive, le poussoir 14 est mobile à l'intérieur du corps 6 jusque dans une position de référence représentée aux figures 6 et 7 dans laquelle les surfaces radiales externes 144 et 146 du poussoir 14 sont respectivement dégagées des surfaces radiales internes 644 et 646 du corps 6. En d'autres termes, dans cette position de référence, les surfaces radiales externes de guidage 144 et 146 sont décalées axialement, le long de l'axe longitudinal A2, des surfaces radiales internes de guidage 644 et 646.

Dans cette position de référence, la partie arrière de la jupe 142 n'est plus engagée dans la partie arrière 64 du corps 6 et la partie avant de la jupe 142 est située plus en avant, le long de l'axe longitudinal A2, que la nervure interne 662.

Dans cette position de référence, les surfaces radiales externes et internes de guidage 144, 146, 644 et 646 peuvent être fabriquées par l'application localisée du liant sur la poudre, sans risque de contact ou de solidarisation intempestive entre ces surfaces même si le jeu de guidage J14 a une épaisseur radiale faible, puisqu'elles sont décalées axialement les unes des autres, le long de l'axe longitudinal A2.

Dans la position de référence, la surface radiale interne de guidage avant 644 est en regard, c'est-à-dire alignée axialement le long de l'axe longitudinal A2 avec, la surface de jonction S15.

Ainsi, un jeu de fabrication J15 existe entre la surface de jonction S15 et la surface radiale interne 644. Ce jeu de fabrication J15 a une épaisseur radiale strictement supérieure à l'épaisseur radiale du jeu de guidage J14.

Par exemple, le jeu de fabrication J15 peut avoir une épaisseur radiale supérieure ou égale à 0.5 mm, de préférence égale à 1 mm.

En d'autres termes, dans la position de référence qui sert à la fabrication additive des éléments 6 et 14 de l'élément de raccord 2, un jeu radial J15 relativement important existe entre le poussoir 14 et la nervure 662, ce jeu radial étant plus épais que le jeu J14 qui sert au guidage en translation du poussoir 14 par rapport au corps 6.

Ceci facilite l'évacuation de grains de poudre qui ne sont pas liés entre eux par le liant au cours de cette fabrication additive, au voisinage des surfaces de guidage.

À cet égard, la présence des cannelures 648, au niveau de la surface radiale interne de guidage avant 644, facilite l'évacuation de la poudre en excès.

La présence des cannelures 650, au niveau de la surface radiale interne de guidage arrière 646, facilite également l'évacuation de la poudre en excès, tout particulièrement dans une variante non-représentée de l'invention où une portion de surface comparable à la surface S15 est engagée dans la surface radiale interne de guidage arrière 646 dans la position de référence du poussoir 14..

Une fois le corps 6 et le poussoir 14 obtenus simultanément par fabrication additive, l'assemblage de l'élément de raccord 2 comprend des étapes successives, effectuées dans l'ordre ci-dessous et consistant à :

- déplacer le poussoir 14 vers une position en butée arrière dans le corps 6 de l'élément de raccord, c'est-à-dire déplacer le poussoir 14 de la position de référence représentée aux figures 6 et 7 à une position comparable à celle des inserts B) des figures 2 à 4 où les surfaces radiales externes de guidage 144 et 146 sont en engagées dans les surfaces radiales internes de guidage 644 et 646;
- pré-positionner les deux mâchoires 10 et 12 dans le boitier 8 ;
- engager le boitier 8 équipé des mâchoires 10 et 12 dans le corps 6, à travers l'ouverture O6, en prenant soin que le bras latéral 105 de la deuxième mâchoire 12 coopère effectivement avec le doigt 148 du poussoir 14, c'est-à-dire en s'assurant que le doigt 148 est bien engagé dans le renfoncement 126 de la deuxième mâchoire ;
- mettre en place et visser les vis 94 et 95 respectivement dans les orifices taraudés 102 et 122 des mâchoires 10 et 12 ;
- monter le ressort de rappel 16 du poussoir 14 dans le volume interne du corps, en appui contre un épaulement 141 du poussoir ;
- mettre en place et visser la canule 4 à l'intérieur du poussoir 14 et du corps 6, grâce au filetage 47 et au taraudage 67, en amenant en appui le ressort 16 contre un épaulement 41 de la canule.

Pour accoupler l'élément de raccord 2 et la durite D2, l'utilisateur prend en main le corps 6 et déplace le levier 106 en direction des surfaces S"6 et S"8 en exerçant le couple C106, ce qui permet d'amener les mâchoires 12 et 14 dans leurs positions de retrait respectives. Il suffit alors d'aligner la durite D2 sur l'axe longitudinal A2 et de déplacer la durite en direction de la canule 4, tout en maintenant le levier 106 appuyé contre le corps 6. Cette opération est facilitée par le fait que la partie avant 42 de la canule 4 dépasse du corps 6 et est pourvue du chanfrein 48.

À un premier stade du rapprochement, la durite D2 entre en contact avec la partie avant 42 la canule et glisse le long de cette partie avant. Ce rapprochement se poursuit jusqu'à ce que la face avant de la durite D2 butte contre un épaulement 43 de la canule qui délimite l'extrémité arrière de la partie avant 42. La durite recouvre alors les nervures périphériques 46. Les mâchoires 10 et 12 sont toujours maintenues en position de retrait par le couple C106 que continue à exercer l'utilisateur sur le levier 106. Elles ne s'opposent donc pas à cet emmanchement.

En libérant le levier 106, c'est-à-dire en cessant d'exercer le couple C106, l'utilisateur laisse les mâchoires revenir en position de serrage, sous l'action du poussoir 14 chargé élastiquement par le ressort 16.

La durite D2 est alors montée sur, raccordée fluidiquement à, et fermement maintenue par rapport à, l'élément de raccord 2.

Pour désaccoupler la durite D2 de l'élément de raccord 2, il suffit de prendre en main le corps 6 et de pousser le levier 106 en direction de ce corps au moyen du couple C106, ce qui ramène les deux mâchoires 10 et 12 dans leurs positions de retrait respectives. L'utilisateur peut alors saisir la durite et la tirer parallèlement à l'axe longitudinal A2, dans un sens d'éloignement vis-à-vis de la canule 4. Comme les mâchoires sont maintenues dans leurs positions de retrait, elles ne s'opposent pas au retrait de la durite D2.

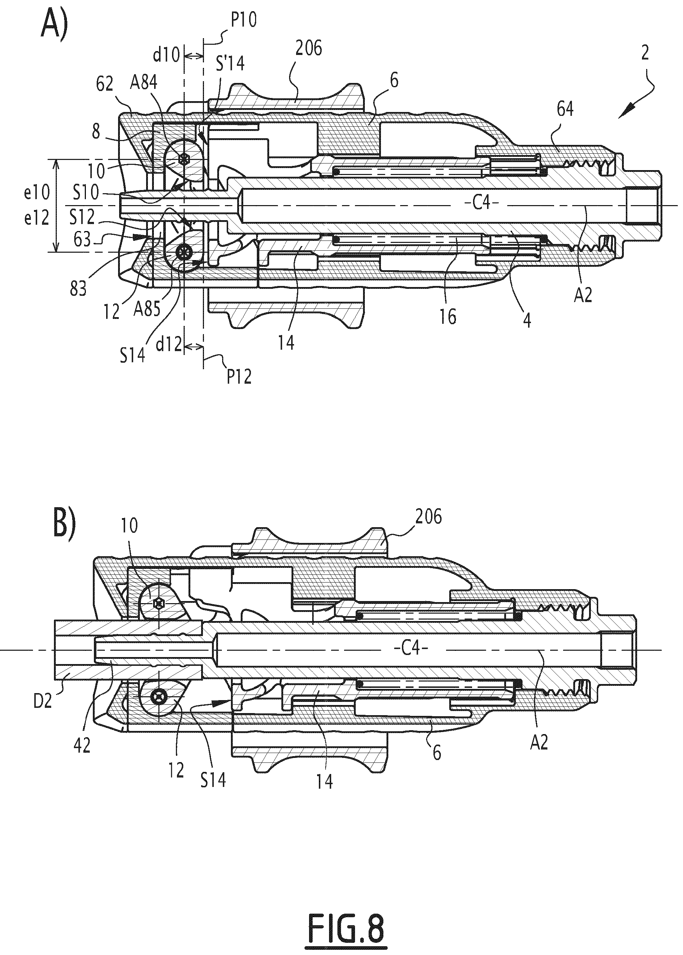

Dans les deuxième, troisième et quatrième modes de réalisation de l'invention représentés aux figures 8 et suivantes, les éléments analogues à ceux du premier mode de réalisation portent des références identiques. Dans ce qui suit, on décrit principalement ce qui distingue ces modes de réalisation du premier mode de réalisation. Si une référence est portée sur l'une des figures 8 et suivantes sans être mentionnée dans la description, ou mentionnée dans la description sans être portée sur une de ces figures, elle correspond au même élément que celui portant la même référence dans le premier mode de réalisation.

L'élément de raccord 2 du deuxième mode de réalisation diffère du précédent en ce que la manoeuvre du poussoir 14, à l'encontre de l'effort élastique exercé par le ressort 16, est effectuée au moyen d'une bague de manoeuvre 206 solidaire, en translation le long de l'axe longitudinal A2 de l'élément de raccord 2, du poussoir 14 qui définit deux surfaces d'appui S14 et S'14 et qui est comparable à celui du premier mode de réalisation.

Dans ce mode de réalisation également, le corps 6 et le poussoir 14 sont avantageusement réalisés ensemble de façon monobloc par fabrication additive, en utilisant une position de référence, comme dans le premier mode de réalisation.

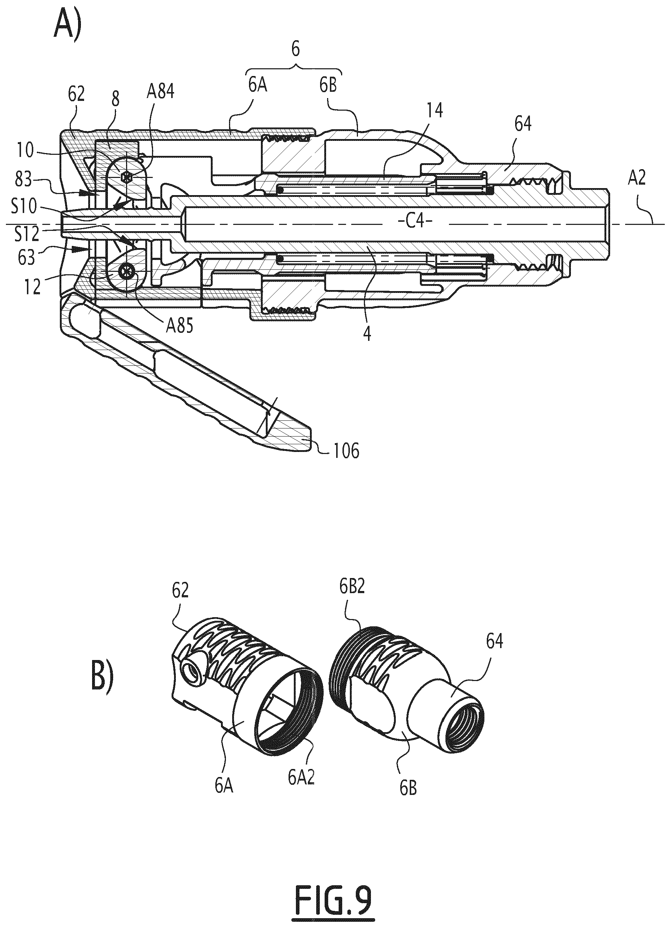

Dans le troisième mode de réalisation représenté à la figure 9, le corps 6 est bipartite est comprend une partie avant 6A et une partie arrière 6B vissées l'une sur l'autre grâce à un taraudage 6A2 prévu sur la partie avant 6A et un filetage 6B2 prévu sur la partie arrière 6B. La partie 6A définit l'extrémité avant 62 du corps 6, alors que la partie arrière définit son extrémité arrière 64.

En variante non représentée de l'invention, le taraudage est prévu sur la partie arrière 6B et le filetage est prévu sur la partie avant 6A.

Dans ce mode de réalisation, il n'est pas obligatoire de réaliser le corps 6 et le poussoir 14 par fabrication additive. Les deux parties 6A et 6B du corps 6 peuvent être montées autour du poussoir 14.

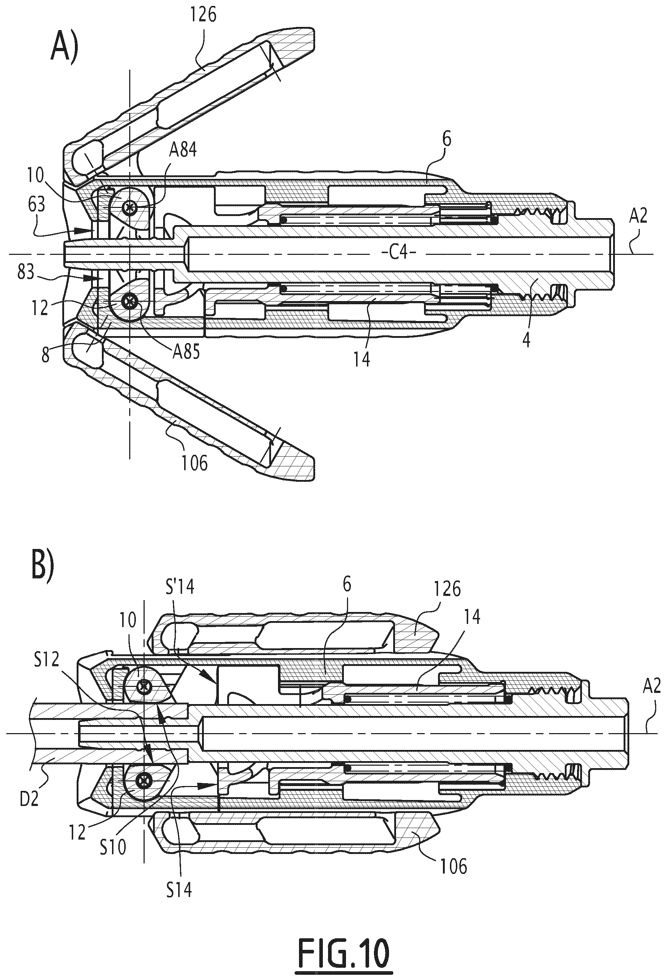

Dans le quatrième mode de réalisation de l'invention représenté à la figure 10, la première mâchoire 10 est commandée par un premier levier 106, alors que la deuxième mâchoire 12 est commandée par un deuxième levier 126. Dans ce cas, ces mâchoires peuvent être manoeuvrées indépendamment l'une de l'autre, respectivement autour du premier axe de rotation A84 et du deuxième axe de rotation A85. Le poussoir 14 peut comprendre deux surfaces d'appui S14 et S'14 analogues à celles du premier mode de réalisation mais ne comprend pas de doigt comparable au doigt 148 du premier mode de réalisation.

Avantageusement, les leviers 106 et 126 sont respectivement monoblocs avec les mâchoires 10 et 12.

Dans ce quatrième mode de réalisation, une fabrication additive du corps 6 et du poussoir 14 est également prévue.

Dans les deuxième à quatrième modes de réalisation, des distances d10, d12 et des écarts radiaux e10, e12 définis comme dans le premier mode de réalisation sont tels que le rapport e10/d10 est strictement supérieur à 1, de même que le rapport e12/d12.

Pour les premier à troisième modes de réalisation et selon une variante non représentée de l'invention, l'élément de raccord peut comprendre une seule mâchoire mobile par rapport au corps. Dans ce cas, un seul rapport, du type du rapport e10/d10, est strictement supérieur à 1.

Lorsqu'un diamètre est mentionné dans la description précède, il correspond à la dimension maximale d'une section d'une ouverture circulaire, transversale à l'axe longitudinal A2. Si cette section n'est pas circulaire, la définition de la dimension maximale en question est adaptée à la géométrie de l'ouverture transversale.

Selon une variante non représentée de l'invention, les longueurs L14 et L16 sont supérieures au longueurs L64 et L66 plus la course C. On a les relations suivantes :

$$L14 \geq L64 + C \qquad \text{(équation 3)}$$

$$L16 \geq L66 + C \qquad \text{(équation 4)}$$

Là encore, grâce à ces relations entre les longueurs L14 et L64, L16 et L66 et la course C, en cours de fonctionnement de l'élément de raccord 2, la surface radiale externe de guidage avant 144 reste en regard de la surface radiale interne de guidage avant 644 et la surface radiale externe de guidage arrière 146 reste en regard de la surface radiale interne de guidage arrière 646, le long de l'axe longitudinal A2.

Toute caractéristique décrite pour un mode de réalisation ou une variante dans ce qui précède peut être mise en oeuvre pour les autres modes de réalisation et variantes décrits précédemment, pour autant que techniquement faisable et tout en restant dans le périmètre des revendications ci-jointes.

## Revendications

1. Elément de raccord (2) pour la connexion d'une canalisation (C2) de fluide à une durite (D2), cet élément de raccord comprenant

   - une canule (4) définissant un passage (C4) de fluide et s'étendant selon un axe longitudinal (A2) de l'élément de raccord, entre une partie avant (42) configurée pour l'emmanchement de la durite et une partie arrière (44) destinée à être raccordée à la canalisation de fluide ;
   - un corps (6) s'étendant selon l'axe longitudinal autour de, et solidairement à, la canule et définissant une embouchure (63) d'accès à la partie avant (42) de la canule ;
   - au moins une première mâchoire (10), mobile en rotation, autour d'un premier axe de rotation (A84), perpendiculaire à, et décalé (d84) de, l'axe longitudinal (A2), entre

     ◦ une position de serrage dans laquelle la première mâchoire (10) presse la durite (D2) contre la partie avant (42) de la canule (4), dans une zone comprise, radialement à l'axe longitudinal, entre l'axe longitudinal et le premier axe de rotation et délimitée, le long de l'axe longitudinal, par un plan frontière (P10), perpendiculaire à l'axe longitudinal et distant du premier axe de rotation d'une première distance (d10), mesurée parallèlement à l'axe longitudinal (A2), qui est non nulle, et
     ◦ une position de retrait dans laquelle la première mâchoire (10) ne presse pas la durite contre la partie avant de la canule ;

   - un organe de manoeuvre (106, 206), accessible depuis l'extérieur du corps (6), pour le déplacement de la première mâchoire, entre sa position de serrage et sa position de retrait,
   **caractérisé en ce que**
   - l'élément de raccord comprend

     ◦ un poussoir (14), logé dans un volume interne (V6) du corps (6) autour de la canule (4) et mobile, en translation le long de l'axe longitudinal (A2), par rapport au corps ;
     ◦ un organe (16) de rappel élastique du poussoir vers une position avancée ;

   - le poussoir (14) est pourvu d'une première surface d'appui (S14) contre la première mâchoire (10) ;
   - la première surface d'appui (S14) du poussoir est configurée pour exercer sur la première mâchoire (10) un effort de déplacement de la première mâchoire de sa position de retrait vers sa position de serrage ;
   - la première surface d'appui (S14) est disposée, par rapport à l'axe longitudinal (A2), à l'opposé du premier axe de rotation (A84) ; et
   - lorsque la première mâchoire (10) est dans sa position de serrage, avec la première surface d'appui (S14) contre la première mâchoire, un premier écart radial (e10), mesuré perpendiculairement à l'axe longitudinal, entre le premier axe de rotation (A84) et un point (P100) de contact de la surface de réception d'effort (S'10) de la première mâchoire (10) et de la première surface d'appui (S14), a une valeur supérieure à la valeur de la première distance (d10).

2. Elément de raccord selon la revendication 1, **caractérisé en ce que** l'organe (16) de rappel élastique du poussoir (14) vers sa position avancée est également un organe de renvoi de la première mâchoire (10) vers sa position de serrage.

EP 4 497 986 B1

3. Elément de raccord selon l'une des revendications précédentes, **caractérisé en ce qu'**un rapport (e10/d10) entre la valeur du premier écart radial (e10) et la valeur de la première distance (d10) est compris entre 1,5 et 10, de préférence entre 4 et 6, de préférence encore égal à 5.

4. Elément de raccord selon l'une des revendications précédentes, **caractérisé en ce que**

- l'élément de raccord (2) comprend également une deuxième mâchoire (12), mobile en rotation, autour d'un deuxième axe de rotation (A85), perpendiculaire à, et décalé (d85) de, l'axe longitudinal (A2), entre

  ◦ une position de serrage dans laquelle la deuxième mâchoire (12) presse la durite (D2) contre la partie avant (42) de la canule (4), dans une zone comprise, radialement à l'axe longitudinal, entre l'axe longitudinal et le deuxième axe de rotation et délimitée, le long de l'axe longitudinal, par un plan frontière (P12), perpendiculaire à l'axe longitudinal et distant du premier axe de rotation d'une deuxième distance (d12), mesurée parallèlement à l'axe longitudinal (A2), qui est non nulle, et
  ◦ une position de retrait dans laquelle la deuxième mâchoire ne presse pas la durite contre la partie avant de la canule ;

- le poussoir (14) comprend une deuxième surface (S'14) d'appui contre la deuxième mâchoire (12) ;
- la deuxième surface d'appui (S'14) du poussoir est configurée pour exercer sur la deuxième mâchoire (12) un effort de déplacement de la deuxième mâchoire de sa position de retrait vers sa position de serrage ;
- la deuxième surface d'appui (S'14) est disposée, par rapport à l'axe longitudinal, à l'opposé du deuxième axe de rotation (A85) ; et
- lorsque la deuxième mâchoire (12) est dans sa position de serrage, avec la deuxième surface d'appui (S'14) contre la deuxième mâchoire, un deuxième écart radial (e12), mesuré parallèlement à l'axe longitudinal, entre le deuxième axe de rotation (A85) et un point (P120) de contact de la surface de réception d'effort (S'12) de la deuxième mâchoire (12) et de la deuxième surface d'appui (S'14), a une valeur supérieure à la valeur de la deuxième distance (d12).

5. Elément de raccord selon la revendication 4, **caractérisé en ce qu'**un rapport (e12/d12) entre la valeur du deuxième écart radial (e12) et la valeur de la deuxième distance (d12) est compris entre 1,5 et 10, de préférence entre 4 et 6, de préférence encore égal à 5.

6. Elément de raccord selon l'une des revendications 4 et 5, **caractérisé en ce que** les valeurs des première et deuxième distances (d10, d12) sont égales et **en ce que** les valeurs des premier et deuxième écarts radiaux (e10, e12) sont égales.

7. Elément de raccord selon l'une des revendications 4 à 6, **caractérisé en ce que** le poussoir (14) comprend un relief (148) configuré pour exercer sur la deuxième mâchoire (12) un effort de déplacement de la deuxième mâchoire, de sa position de serrage vers sa position de retrait.

8. Elément de raccord selon l'une des revendications précédentes, **caractérisé en ce que** l'organe de manoeuvre de la première mâchoire (10) est un levier (106) solidaire de la première mâchoire.

9. Elément de raccord selon l'une des revendications 1 à 7, **caractérisé en ce que** l'organe de manoeuvre de la première mâchoire (10) est une bague (206) mobile axialement, le long de l'axe longitudinal (A2), par rapport au corps (6).

10. Procédé de fabrication d'un élément de raccord selon l'une des revendications précédentes, **caractérisé en ce que**

- le corps (6) est pourvu d'au moins une surface radiale interne de guidage (644, 646) ;
- le poussoir (14) est pourvu d'au moins une surface radiale externe de guidage (144, 146) configurée pour coopérer par engagement avec une surface radiale interne de guidage du corps pour guider le poussoir lors de ses déplacements selon l'axe longitudinal (A2) ;
- le poussoir (14) est mobile, dans le passage du corps, jusqu'à une position de référence, dans laquelle la ou chaque surface radiale externe de guidage (144, 146) du poussoir est dégagée de toute surface radiale interne de guidage (644, 646) du corps (6) et réciproquement ;
- le procédé comprend une étape consistant à

a) réaliser par fabrication additive le corps (6) et le poussoir (14) simultanément, alors que le poussoir est dans sa position de référence.

**11.** Procédé selon la revendication 10 de fabrication d'un élément de raccord selon l'une des revendications 4 à 7, **caractérisé en ce qu'**il comprend des étapes successives postérieures à l'étape a) et consistant à

b) déplacer le poussoir (14) dans le corps (6) de l'élément de raccord (2) vers une position où les surfaces radiales externes de guidage (144, 146) sont engagées dans les surfaces radiales internes de guidage (644, 646) ;

c) pré-positionner les deux mâchoires (10, 12) dans un boitier (8) ;

d) engager le boitier (8) équipé des mâchoires (10, 12) dans le corps (6), à travers une ouverture (O6) ménagée dans le corps ;

e) mettre en place et visser des vis (94, 95) respectivement dans des paliers (842, 852) du boîtier (8) et dans des orifices taraudés (102, 122) des mâchoires (10, 12) de telle sorte que ces vis soient alignées sur le premier axe de rotation (A84) et sur le deuxième axe de rotation (A85);

f) monter l'organe (16) de rappel élastique du poussoir (14) dans le volume interne (V6) du corps, en appui contre le poussoir ;

g) mettre en place la canule (4) à l'intérieur du poussoir (14) et du corps (6), en amenant l'organe (16) de rappel élastique en appui contre la canule.

**Patentansprüche**

**1.** Verbindungselement (2) zum Verbinden einer Fluidleitung (C2) mit einem Schlauch (D2), das Verbindungselement umfassend

- eine Kanüle (4), die einen Fluiddurchgang (C4) definiert und sich entlang einer Längsachse (A2) des Verbindungselements zwischen einem vorderen Abschnitt (42), der zum Einstecken des Schlauchs konfiguriert ist, und einem hinteren Abschnitt (44), der dazu bestimmt ist, mit der Fluidleitung verbunden zu werden, erstreckt;

- einen Körper (6), der sich entlang der Längsachse um die Kanüle erstreckt und fest damit verbunden ist, und eine Mündung (63) für den Zugang zu dem vorderen Abschnitt (42) der Kanüle definiert;

- mindestens eine erste Klemmbacke (10), die um eine erste Drehachse (A84), die senkrecht zu und versetzt (d84) von der Längsachse (A2) ist, drehbar beweglich ist, zwischen

∘einer Klemmstellung, in der die erste Klemmbacke (10) den Schlauch (D2) gegen den vorderen Abschnitt (42) der Kanüle (4) drückt, in einem Bereich, der radial zu der Längsachse zwischen der Längsachse und der ersten Drehachse ist und entlang der Längsachse durch eine Grenzebene (P10) begrenzt wird, die senkrecht zu der Längsachse ist und von der ersten Drehachse um einen ersten Abstand (d10), gemessen parallel zu der Längsachse (A2), entfernt ist, der ungleich Null ist, und

∘einer zurückgezogene Stellung, in der die erste Klemmbacke (10) den Schlauch nicht gegen den vorderen Abschnitt der Kanüle drückt;

- ein von außerhalb des Körpers (6) zugängliches Betätigungsorgan (106, 206) zum Bewegen der ersten Klemmbacke zwischen ihrer Klemmstellung und ihrer zurückgezogenen Stellung, **dadurch gekennzeichnet, dass**

- das Verbindungselement Folgendes umfasst

∘einen Stößel (14), der in einem inneren Volumen (V6) des Körpers (6) um die Kanüle (4) untergebracht und entlang der Längsachse (A2) in Bezug auf den Körper translatorisch beweglich ist;

∘ ein elastisches Rückholorgan (16) des Stößels in eine vorgeschobene Stellung;

- wobei der Stößel (14) mit einer ersten Anlagefläche (S14) an der ersten Klemmbacke (10) versehen ist;

- die erste Anlagefläche (S14) des Stößels konfiguriert ist, um eine Kraft auf die erste Klemmbacke (10) auszuüben, um die erste Klemmbacke aus ihrer zurückgezogenen Stellung in ihre Klemmstellung zu bewegen;

- die erste Anlagefläche (S14) in Bezug auf die Längsachse (A2) gegenüber der ersten Drehachse (A84) angeordnet ist; und

- wenn die erste Klemmbacke (10) in ihrer Klemmstellung ist, mit der ersten Anlagefläche (S14) gegen die erste Klemmbacke, eine erste radiale Abweichung (e10), gemessen senkrecht zu der Längsachse, zwischen der ersten Drehachse (A84) und einem Kontaktpunkt (P100) der Kraftaufnahmefläche (S'10) der ersten Klemmbacke (10) und der ersten Anlagefläche (S14) einen Wert aufweist, der größer ist als der Wert des ersten Abstands (d10).

2. Verbindungselement nach Anspruch 1, **dadurch gekennzeichnet, dass** das elastische Rückholorgan (16) des Stößels (14) in seine vorgeschobene Stellung auch ein Umlenkorgan der ersten Klemmbacke (10) in ihre Klemmstellung ist.

3. Verbindungselement nach einem der vorherigen Ansprüche, **dadurch gekennzeichnet, dass** ein Verhältnis (e10/d10) zwischen dem Wert der ersten radialen Abweichung (e10) und dem Wert des ersten Abstands (d10) zwischen 1,5 und 10, vorzugsweise zwischen 4 und 6, bevorzugter gleich 5, ist.

4. Verbindungselement nach einem der vorherigen Ansprüche, **dadurch gekennzeichnet, dass**

   - das Verbindungselement (2) auch eine zweite Klemmbacke (12) umfasst, die um eine zweite Drehachse (A85), die senkrecht zu und versetzt (d85) von der Längsachse (A2) ist, drehbar beweglich ist zwischen

     ◦ einer Klemmstellung, in der die zweite Klemmbacke (12) den Schlauch (D2) gegen den vorderen Abschnitt (42) der Kanüle (4) drückt, in einem Bereich, der radial zu der Längsachse zwischen der Längsachse und der zweiten Drehachse ist und entlang der Längsachse durch eine Grenzebene (P12) begrenzt wird, die senkrecht zu der Längsachse ist und von der ersten Drehachse um einen ersten Abstand (d12), gemessen parallel zu der Längsachse (A2), entfernt ist, der ungleich Null ist, und
     ◦ einer zurückgezogene Stellung, in der die zweite Klemmbacke den Schlauch nicht gegen den vorderen Abschnitt der Kanüle drückt;

   - der Stößel (14) eine zweite Anlagefläche (S'14) an der zweiten Klemmbacke (12) umfasst;
   - die zweite Anlagefläche (S'14) des Stößels konfiguriert ist, um eine Kraft auf die zweite Klemmbacke (12) auszuüben, um die zweite Klemmbacke aus ihrer zurückgezogenen Stellung in ihre Klemmstellung zu bewegen;
   - die zweite Anlagefläche (S'14) in Bezug auf die Längsachse gegenüber der zweiten Drehachse (A85) angeordnet ist; und
   - wenn die zweite Klemmbacke (12) in ihrer Klemmstellung ist, mit der zweiten Anlagefläche (S'14) gegen die zweite Klemmbacke, eine zweite radiale Abweichung (e12), gemessen parallel zu der Längsachse, zwischen der zweiten Drehachse (A85) und einem Kontaktpunkt (P120) der Kraftaufnahmefläche (S'12) der zweiten Klemmbacke (12) und der zweiten Anlagefläche (S'14) einen Wert aufweist, der größer ist als der Wert des zweiten Abstands (d12).

5. Verbindungselement nach Anspruch 4, **dadurch gekennzeichnet, dass** ein Verhältnis (e12/d12) zwischen dem Wert der zweiten radialen Abweichung (e12) und dem Wert des zweiten Abstands (d12) zwischen 1,5 und 10, vorzugsweise zwischen 4 und 6, bevorzugter gleich 5, ist.

6. Verbindungselement nach einem der Ansprüche 4 und 5, **dadurch gekennzeichnet, dass** die Werte des ersten und des zweiten Abstands (d10, d12) gleich sind und dass die Werte der ersten und der zweiten radialen Abweichung (e10, e12) gleich sind.

7. Verbindungselement nach einem der Ansprüche 4 bis 6, **dadurch gekennzeichnet, dass** der Stößel (14) ein Relief (148) umfasst, das konfiguriert ist, um eine Kraft auf die zweite Klemmbacke (12) auszuüben, um die zweite Klemmbacke von ihrer Klemmstellung in ihre zurückgezogene Stellung zu bewegen.

8. Verbindungselement nach einem der vorherigen Ansprüche, **dadurch gekennzeichnet, dass** das Betätigungsorgan der ersten Klemmbacke (10) ein Hebel (106) ist, der fest mit der ersten Klemmbacke verbunden ist.

9. Verbindungselement nach einem der Ansprüche 1 bis 7, **dadurch gekennzeichnet, dass** das Betätigungsorgan der ersten Klemmbacke (10) ein Ring (206) ist, der axial entlang der Längsachse (A2) in Bezug auf den Körper (6) beweglich ist.

10. Verfahren zur Herstellung eines Verbindungselements nach einem der vorherigen Ansprüche, **dadurch gekennzeichnet, dass**

    - der Körper (6) mit mindestens einer inneren radialen Führungsfläche (644, 646) versehen ist;

- der Stößel (14) mit mindestens einer äußeren radialen Führungsfläche (144, 146) versehen ist, die konfiguriert ist, um durch Eingriff mit einer inneren radialen Führungsfläche des Körpers zusammenzuwirken, um den Stößel bei seinen Bewegungen entlang der Längsachse (A2) zu führen;

- der Stößel (14) in dem Durchgang des Körpers in eine Referenzstellung bewegbar ist, in der die oder jede radial äußere Führungsfläche (144, 146) des Stößels frei von jeder radial inneren Führungsfläche (644, 646) des Körpers (6) ist und umgekehrt;

- das Verfahren einen Schritt umfasst, der aus Folgendem besteht

a) gleichzeitiges Herstellen, durch additive Fertigung, des Körpers (6) und des Stößels (14), während der Stößel in seiner Referenzstellung ist.

11. Verfahren nach Anspruch 10 zur Herstellung eines Verbindungselements nach einem der Ansprüche 4 bis 7, **dadurch gekennzeichnet, dass** es aufeinanderfolgende Schritte nach Schritt a) umfasst, die aus Folgendem bestehen

b) Bewegen des Stößels (14) in dem Körper (6) des Verbindungselements (2) in eine Stellung, in der die äußeren radialen Führungsflächen (144, 146) mit den inneren radialen Führungsflächen (644, 646) in Eingriff sind;

c) Vorpositionieren der zwei Klemmbacken (10, 12) in einem Gehäuse (8);

d) Einsetzen des mit den Klemmbacken (10, 12) ausgestatteten Gehäuses (8) in den Körper (6) durch eine Öffnung (06) in dem Körper;

e) Einsetzen und Einschrauben von Schrauben (94, 95) jeweils in Lager (842, 852) des Gehäuses (8) und in Gewindeöffnungen (102, 122) der Klemmbacken (10, 12), sodass diese Schrauben auf die erste Drehachse (A84) und auf die zweite Drehachse (A85) ausgerichtet sind;

f) Montieren des elastischen Rückholorgans (16) des Stößels (14) in dem inneren Volumen (V6) des Körpers in Anlage an den Stößel;

g) Einsetzen der Kanüle (4) in das Innere des Stößels (14) und des Körpers (6), indem das elastische Rückholorgan (16) in Anlage an die Kanüle gebracht wird.

## Claims

1. A coupling element (2) for connecting a fluid pipe (C2) to a hose (D2), the coupling element comprising

- a cannula (4) defining a passage (C4) for fluids and extending along a longitudinal axis (A2) of the coupling element, between a front part (42) configured for the fitting on of the hose and a rear part (44) to be coupled to the fluid pipe (C2);

- a body (6) extending longitudinally about the cannula and secured thereto, defining a mouth (63) for access to the front part (42) of the cannula;

- at least one first jaw (10) movable in rotation about a first axis of rotation (A84) perpendicular to and offset (D84) from the longitudinal axis (A2) between

◦ a clamping position wherein the first jaw (10) presses the hose (D2) against the front part (42) of the cannula (4) in a zone comprised, radially to the longitudinal axis between the longitudinal axis and the first axis of rotation and bounded along the longitudinal axis by a boundary plane (P10), perpendicular to the longitudinal axis and spaced from the first axis of rotation by a first distance (d10), measured parallel to the longitudinal axis (A2), which is non-zero, and

◦ a retracted position wherein the first jaw (10) does not press the hose against the forward portion of the cannula;

- a manoeuvring member (106, 206), accessible from outside the body (6), for moving the first jaw between the clamping position thereof and the retracted position thereof,

**characterized in that**

- the coupling element comprises

◦ a pusher (14), housed in an internal volume (V6) of the body (6) around the cannula (4) and movable, in translation along the longitudinal axis (A2), relative to the body;

◦ a member (16) for elastic return of the pusher toward the forward position thereof;

- the pusher (14) is provided with a first surface (S14), bearing against the first jaw (10);
- the first bearing surface (S14) of the pusher is configured to exert on the first jaw (10) a force for moving the first jaw from the released position thereof to the clamping position thereof;
- the first bearing surface (S14) is arranged opposite the first axis of rotation (A84) in relation to the longitudinal axis (A2); and
- when the first jaw (10) is in the clamping position thereof, with the first bearing surface (S14) against the first jaw, a first radial gap (e10), measured perpendicular to the longitudinal axis, between the first axis of rotation (A84) and a contact point (P100) of the force-receiving surface (S'10) of the first jaw (10) and the first bearing surface (S14) has a value greater than the value of the first distance (D10).

2. The coupling element according to claim 1, **characterized in that** the member (16) for elastically returning the pusher (14) to the advanced position thereof is also a member for returning the first jaw (10) to the clamping position thereof.

3. The coupling element according to one of the preceding claims, **characterized in that** a ratio (e10/d10) between the value of the first radial gap (e10) and the value of the first distance (d10) is comprised between 1.5 and 10, preferably between 4 and 6, more preferably equal to 5.

4. The coupling element according to any of the preceding claims, **characterized in that**

  - the first coupling element (2) further comprises a second jaw (12) movable in rotation about a second axis of rotation (A85) perpendicular to and offset (d85) from the longitudinal axis (A2), between

    ○ a clamping position wherein the second jaw (12) presses the hose (D2) against the front part (42) of the cannula (4) in a zone comprised, radially to the longitudinal axis between the longitudinal axis and the second axis of rotation and bounded along the longitudinal axis by a boundary plane (P12), perpendicular to the longitudinal axis and spaced from the first axis of rotation by a second distance (d12), measured parallel to the longitudinal axis (A2), which is non-zero, and
    ○ a retracted position wherein the second jaw does not press the hose against the front part of the cannula;

  - the pusher (14) comprising a second surface (S'14), bearing against the second jaw (12);
  - the second bearing surface (S'14) of the pusher is configured to exert on the second jaw (12) a force for moving the second jaw from the retracted position thereof to the clamping position thereof;
  - the second bearing surface (S'14) is arranged opposite the second of rotation (A85) in relation to the longitudinal axis; and
  - when the second jaw (12) is in the clamping position thereof, with the second bearing surface (S'14) against the second jaw, a second radial gap (e12), measured perpendicular to the longitudinal axis, between the first axis of rotation (A85) and a contact point (P120) of the force-receiving surface (S'12) of the second jaw (12) and the second bearing surface (S'14) has a value greater than the value of the second distance (d12).

5. The coupling element according to claim 4, **characterized in that** a ratio (e12/d12) between the value of the second radial gap (e12) and the value of the second distance (d12) is comprised between 1.5 and 10, preferably between 4 and 6, more preferably equal to 5.

6. The coupling element according to one of claims 4 and 5, **characterized in that** the values of the first and second distances (d10, d12) are equal and **in that** the values of the first and second radial gaps (e10, e12) are equal.

7. The coupling element according to one of claims 4 to 6, **characterized in that** the pusher (14) comprises a relief (148) configured to exert on the second jaw (12) a force for moving the second jaw from the clamping position thereof to the retracted position thereof.

8. The coupling element according to one of the preceding claims, **characterized in that** the manoeuvring member of the first jaw (10) is a lever (106) secured to the first jaw.

9. The coupling element according to one of claims 1 to 7, **characterized in that** the manoeuvring member of the first jaw (10) is a ring (206) which is axially movable, along the longitudinal axis (A2), relative to the body (6).

10. A manufacturing method for a coupling element according to one of the preceding claims, **characterized in that**

- the body (6) is provided with at least one inner radial guiding surface (644, 646);
- the pusher (14) is provided with at least one outer radial guiding surface (144, 146) configured to engage the inner radial guiding surface to guide the pusher during the movements thereof along the longitudinal axis (A2);
- the pusher (14) is movable, inside the passage of the body, to a reference position, wherein the or each outer radial guiding surface (144, 146) of the pusher is disengaged from any inner radial guiding surface (644, 646) of the body (6) and vice versa;
- the method comprises a step consisting of

    a) making simultaneously, by additive manufacturing, the body (6) and the pusher (14), while the pusher is in the reference position thereof.

11. The method according to claim 10 for manufacturing a coupling element according to one of claims 4 to 7, **characterized in that** it comprises successive steps subsequent to step a) and consisting in

    b) moving the pusher (14) in the body (6) of the coupling element (2) to a position where the radial outer guiding surfaces (144, 146) are engaged with the inner radial guiding surface (644, 646);
    c) pre-positioning the two jaws (10,12) in a housing (8);
    d) engaging the housing (8) equipped with jaws (10, 12) in the body (6) through an opening (O6) provided in the body;
    e) fitting and screwing screws (94, 95) in bearings (842, 852) of the housing (8) and in tapped orifices (102, 122) of the jaws (10, 12), respectively, in such a way that the screws are aligned with the first axis of rotation (A84) and with the second axis of rotation (A85);
    f) mounting the member (16) for elastically returning the pusher (14) in the internal volume (V6) of the body, bearing against the pusher;
    g) fitting the cannula (4) inside the pusher (14) and the body (6), bringing the elastic return member (16) into abutment against the cannula.

FIG.1

FIG.2

A)

B)

FIG.3

A)

B)

FIG.4

FIG.5

## FIG.6

FIG.7

## FIG.8

A)

B)

# FIG.9

FIG.10

**EP 4 497 986 B1**